# EUROPEAN PATENT APPLICATION

(11) **EP 3 884 860 A1**
(43) Date of publication of application: **29.09.2021**
(21) Application number: 20165842.4
(22) Date of filing: 26.03.2020
(51) Int. Cl.: A61B 5/0452, A61B 5/0456, A61B 5/00, A61N 1/37, A61N 1/36

(54) **IMPLANTABLE MEDICAL DEVICE FOR STIMULATING A HUMAN OR ANIMAL HEART**

(71) Applicant: BIOTRONIK SE & Co. KG, 12359 Berlin (DE)
(72) Inventor: BECKER, Frank, 10409 Berlin (DE)
(74) Representative: Biotronik Corporate Services SE

(57) **Abstract**

The invention relates to an implantable medical device for stimulating a human or animal heart, comprising a processor, a memory unit, a stimulation unit configured to stimulate the His bundle of a human or animal heart, and a detection unit configured to detect an electrical signal at the His bundle of the same heart. The memory unit comprises a computer-readable program that causes the processor to perform, amongst others, the following steps when executed on the processor: classifying two consecutive first peaks (3,4; 4,5) as R waves if an interval (T1, T2) between these two consecutive first peaks (3,4; 4,5) is bigger than 80 % and smaller than 120 % of the mean interval (TM); and adjusting a detection threshold (2) of the detection unit so as to allow the detection unit to exclusively detect first peaks (3, 4, 5, 6, 7) in the electrical signal (1) of the human or animal heart.

## Description

The present invention relates to an implantable medical device for stimulating a human or animal heart according to the preamble of claim 1, to a method for determining an R wave detection threshold of a His electrode channel of such an implantable medical device according to the preamble of claim 10, and to a computer program product for carrying out such method according to the preamble of claim 11.

Implantable medical devices for stimulating a human or animal heart, such as pacemakers, have been known for a long time. They can perform different functions. Different stimulation programs can be carried out by an appropriate pacemaker to restore the treated heart to a normal state. Pacemakers are also known to stimulate the His bundle.

The His bundle is a bundle of specific heart muscle cells that is part of the cardiac conduction system. The His bundle is located distally of the atrioventricular node towards the apex of the heart.

Often conventional pacemakers and implantable defibrillators are used for His bundle stimulation. A stimulation output of such a conventional device is connected to a His-bundle electrode. The conventional devices are then typically adapted to the requirements of His bundle stimulation within the available parameter value ranges, making compromises.

There exist also specific devices adapted for His bundle pacing, wherein a detecting (sensing) and stimulation electrode is not implanted into the ventricle of the human or animal heart be treated, but rather at or near to the His bundle of the heart. Such use of a His bundle electrode enables a particularly physiologic stimulation of the human or animal heart. However, it is significantly more difficult to safely detect an intrinsic stimulation of the right ventricle (RV) of the human or animal heart at the His bundle in comparison to a conventional electrode implantation site (i.e., within the right ventricle). This is due to the fact that R wave signals are much smaller at the His bundle than in the right ventricle and that stimulation signals of the His bundle itself are additionally detected. Furthermore, there is not only a single stimulation threshold in case of His bundle pacing. Rather, up to three stimulation thresholds are known, namely, the so-called selective stimulation threshold, the so-called non-selective stimulation threshold and the so-called myocardium stimulation threshold. The optimum stimulation amplitude is determined on the basis of these stimulation thresholds.

US 8,565,880 B2 describes step-down and step-up tests for finding an appropriate His bundle capture threshold. This US patent further describes a verification of His bundle capture according to electrocardiogram (ECG) signal characteristics such as amplitude, pulse width and morphology.

It is an object of the present invention to provide an implantable medical device for stimulating a human or animal heart that enables a more appropriate His bundle pacing than implantable medical devices known from prior art.

This object is achieved with an implantable medical device for stimulating a human or animal heart having the claim elements of claim 1. Such an implantable medical device comprises a processor, a memory unit, a stimulation unit and a detection unit. The stimulation unit is specifically configured to stimulate the His bundle of the human or animal heart. The detection unit is specifically configured to detect an electrical signal at His bundle of the same heart. Typically, both the stimulation unit and the detection unit are connected to one and the same His bundle electrode that is implanted at or very close to the His bundle of the human or animal heart to be stimulated.

According to an aspect of the presently claimed invention, the memory unit comprises a computer-readable program that causes the processor to perform the steps explained in the following when being executed on the processor.

In a first step, an electrical signal (e.g., an ECG) of the human or animal heart is detected with the detection unit. This electrical signal is detected at the His bundle of the human or animal heart to be treated.

Subsequently, first peaks within the electrical signal are identified. In doing so, one assumes that the first peaks are R waves of the electrical signal. This, however, needs to be confirmed by the measures explained in the following.

In a further step, an interval between two consecutive first peaks is determined. Based on a number of such intervals, a mean interval between two consecutive first peaks in each case is determined for first number of pairs of consecutive first peaks. The first number can, e.g., be a number between 1 and 100, in particular between 2 and 95, in particular between 3 and 90, in particular between 4 and 85, in particular between 5 and 80, in particular between 6 and 75, in particular between 7 and 70, in particular between 8 and 65, in particular between 9 and 60, in particular between 10 and 55, in particular between 11 and 50, in particular between 12 and 45, in particular between 13 and 40, in particular between 14 and 35, in particular between 15 and 30, in particular between 20 and 25.

Depending on the determined interval between two consecutive first peaks, these first peaks are then classified as R waves. To be more precise, two consecutive first peaks are classified as R waves if the interval between these first peaks lies within a range of more than 80 % but less than 120 % of the mean interval. If the interval between two first peaks is bigger than 120 % of the mean interval or lower than 80 % of the mean interval, it is considered that one of the first peaks is an R wave and the other of the first peaks is a ventricular extrasystole or a further cardiac signal other than an R wave.

After at least some of the first peaks have been successfully identified as R waves, the detection threshold of the detection unit is adjusted so as to be able to also detect R waves having a smaller amplitude than the already detected R waves, but to detect at the same time no other cardiac signals than R waves in the analyzed electrical signal of the heart to be treated. For this purpose, the detection threshold of the detection unit is adjusted to allow an exclusive detection of R waves in the electrical signal of the human or animal heart. This adjustment is done by performing the steps explained in the following.

First, an initial detection threshold is defined. In doing so, the detection threshold corresponds in a first variant to an amplitude of a single first peak that has already been successfully classified as R wave. In a second variant, the detection threshold corresponds to a mean amplitude of a plurality of first peaks that have already been successfully classified as R waves. Thus, in this step a rather non-sensitive detection threshold is defined that is appropriate to detect future R waves if the R wave amplitude does not significantly decrease with respect to the R waves already detected within the electrical signal of the human or animal heart.

Subsequently, the detection threshold is iteratively lowered until at least one second peak is identified within the electrical signal. It is to be noted that the second peak is a physiologic different peak than the first peak. For such identification or classification of the second peak, it is necessary that an interval between the at least one second peak and an adjacent first peak, preferably the preceding first peak, is in a range of the length of an absolute refractory period of the heart. According to an embodiment, the second peak is identified within the electrical signal if an interval between the at least one second peak and an adjacent first peak, preferably the preceding first peak, is smaller than or equal to 80% of the mean interval. Typically, only a P wave or a signal of the His bundle activity can be located within such a small time interval to an R wave. In an embodiment, care is taken that the at least one second peak is observed prior to an adjacent first peak within an interval being in a range of the length of an absolute refractory period of the heart.

According to an embodiment, the detection threshold is iteratively decreased until at least one second peak is identified within the electrical signal, wherein the second peak has an amplitude which is lower than the amplitude of the single first peak that has been successfully classified as R wave or the mean amplitude of the plurality of first peaks that have been successfully classified as R wave. A second peak having said lower amplitude than the first peak, or a lower amplitude than a mean amplitude of multiple first peaks, is typically either a P-wave or a signal of the HIS bundle activity. For instance, the amplitude of the second peak may be lower or equal to 75 %, or 50 %, or 40 %, or 30 %, or 20 %, or 10 % of the first peak or of the mean amplitude of multiple first peaks.

If the detection threshold would be left at this level, not only R waves, but also other cardiac signals such as a P wave or a His bundle activity would be observed. However, for proper functioning of the implantable medical device, it is particularly important to detect only R waves in order to have positive knowledge on ventricular activity of the human or animal heart to be stimulated. Therefore, the detection threshold is subsequently increased by a predetermined factor. This increase causes that only first peaks (i.e., suspected R waves) but no second peaks can be identified within the electrical signal any longer. Expressed in other words, the detection threshold is lowered to such a level that also small ventricular activities having a small amplitude of the R wave can be securely detected, wherein other cardiac signals that could generally be detected at the His bundle are not detected. Then, the sensing or detection threshold is set to such a level that enables secure and reliable operation of the implantable medical device, wherein a medical relevance of the detected signals is ensured.

During operation of the implantable medical device, the device evaluates the measured electric signal with respect to an occurrence of an R wave. If an R wave (or, alternatively, a ventricular extrasystole) is detected, the implantable medical device needs not to perform His bundle pacing. If, in contrast, no intrinsic R wave can be measured, the implantable medical device needs to perform a His bundle pacing by emitting a stimulation pulse by the stimulation unit to achieve a ventricular contraction of the human or animal heart to be treated.

While it is particularly difficult to securely detect an electrical signal that can be safely attributed to an R wave of an electric signal of the human or animal heart with a His bundle electrode, the presently claimed invention provides a particularly simple and reliable methods to reliably detect R waves in an electric cardiac signal and to distinguish the R wave peaks from other cardiac peaks being present in the electric signal from the human or animal heart.

In an embodiment, the computer-readable program causes the processor not to use an interval between two consecutive first peaks for determining the mean interval between two consecutive first peaks if the interval is too small with respect to an expectancy value. In the present case, the expectancy value is given by at least two (in particular 2 to 10, in particular 3 to 9, in particular 4 to 8, in particular 5 to 7) other intervals between two consecutive first peaks in each case. If the measured interval is either smaller than or equal to 80 % of at least two other intervals between two consecutive peaks or bigger than or equal to 120 % of at least two other intervals between two consecutive peaks, this indicates that one of the peaks limiting the interval is not an R wave, but rather another cardiac peak, such as a ventricular extrasystole. In such a case, the interval should not be used for determining the mean interval in order to not distort the calculation of the mean interval.

In an embodiment, the interval is not used for determining the mean interval if the interval is smaller than or equal to 75 %, in particular 70 %, in particular 65 %, in particular 60 %, in particular 55 %, in particular 50 % of at least two other intervals between two consecutive first peaks.

In an embodiment, the interval is not used for determining the mean interval if the interval is bigger than or equal to 125 %, in particular 130%, in particular 135 %, in particular 140 %, in particular 145 %, in particular 150 % of at least two other intervals between two consecutive first peaks.

In an embodiment, the computer-readable program causes the processor not to use two consecutive intervals between two consecutive first peaks in each case for determining the mean interval between two consecutive first peaks, if these two consecutive intervals do not comply with certain expectancy values. To be more precise, the two consecutive intervals are not used for determining the mean interval if the first of the two consecutive intervals is smaller than or equal to 80 %, in particular 75 %, in particular 70 %, in particular 65 %, in particular 60 %, in particular 55 %, in particular 50 % of at least two other intervals between two consecutive first peaks. Furthermore, at the same time, it is necessary that the second of the two consecutive intervals is bigger than or equal to 120 %, in particular 125%, in particular 130%, in particular 135%, in particular 140%, in particular 145%, in particular 150% of at least two other intervals between two consecutive first peaks in order to discard the two consecutive intervals for determining the mean interval. If both conditions are met, it is very likely that the two consecutive intervals do not belong to two intervals between two R waves of the electric signal of the human or animal heart, but rather to an interval between an R wave and a ventricular extrasystole as well as to an interval between the same ventricular extrasystole and the next R wave. In such a case, considering the intervals could distort the calculation of the mean interval so that discarding the intervals for the purpose of calculating the mean interval between two consecutive first peaks enhances the reliability of the overall process for determining the mean interval between two consecutive first peaks.

In an embodiment, the computer-readable program causes the processor to continuously update the mean interval between two consecutive first peaks. In doing so, even small trends towards an overall decreased amplitude of the R wave or towards an overall increased amplitude of the R wave can be considered so that the starting point for adjusting the detection threshold of the detection unit is always at or close to a physiologic relevant level.

In an embodiment, the computer-readable program causes the processor not to use an interval between two consecutive first peaks, if either of the following two conditions is met. The first condition is an interval being smaller than or equal to 80 %, in particular 75 %, in particular 70 %, in particular 65 %, particular 60 %, in particular 55 %, in particular 50 % of the current mean interval between two consecutive first peaks. The second condition is an interval being bigger than or equal to 120 %, in particular 125 %, in particular 130 %, in particular 135 %, in particular 140 %, in particular 145 %, in particular 150 % of the current mean interval between two consecutive first peaks. The term "current mean interval" denotes in this context the mean interval between two consecutive first peaks before the updating process.

In an embodiment, the predetermined factor by which the detection threshold is increased after an additional cardiac peak other than an R wave has been identified in the electric cardiac signal serves for an increase of the detection threshold to a level corresponding to a level prior to the last decrease of the detection threshold during the iterative decrease of the detection threshold. Thus, the detection threshold is set to the last value and which only R wave signals were detected, but no other cardiac signals. It is possible to apply an additional safety margin, e.g. by applying an additional factor that can be chosen like the factor explained in the following embodiment.

In an embodiment, the predetermined factor by which the detection threshold is increased after an additional cardiac peak other than an R wave has been identified in the electric cardiac signal is at least 1.1, in particular at least 1. 2, in particular at least 1.3, in particular at least 1.4, in particular at least 1.5, in particular at least 1.6, in particular at least 1.7, in particular at least 1.8, in particular at least 1.9, in particular at least 2.0 of an absolute voltage of the detection threshold. In an embodiment, the factor lies between 1.1 and 2.0, in particular between 1.2 and 1.9, in particular between 1.3 and 1.8, in particular between 1.4 and 1.7, in particular between 1.5 and 1.6 of an absolute voltage of the detection threshold. Thus, in this embodiment, a safety margin between 10 % and 100 % is added to the detected lowest detection threshold in order to set the detection threshold for further operation of the implantable medical device. The absolute voltage of the detection threshold lies typically in a range between 1 mV and 25 mV, in particular between 2 mV and 22.5 mV, in particular between 3 mV and 20 mV, in particular between 4 mV and 17.5 mV, in particular between 5 mV and 15 mV, in particular between 7.5 mV and 12.5 mV, in particular between 8 mV and 10 mV. Thus, to give an example, the determined minimum detection threshold is increased by the modification with the predetermined factor by, e.g., 0.1 mV to 2.5 mV.

In an embodiment, the computer-readable program causes the processor to stop the iterative lowering of the detection threshold if a first number of second peaks is identified within the electrical signal in a second number of consecutive heart cycles. In this context, the first number is smaller than the second number. To give an example, the first number may be 1, 2, 3, 4, 5, 6, 7, 8, 9, or 10. Likewise, the second number may be 3, 4, 5, 6, 7, 8, 9, 10, 11, 12, 13, 14, or 15. If one second peak is identified in three consecutive heart cycles (a heart cycle starts at the mid of an R wave and ends at the mid of the next adjacent R wave), altogether four cardiac peaks (three first peaks and one second peak) are detected within these three heart cycles. Then, in an embodiment, a sufficient condition is met to stop the iterative lowering of the detection threshold. In another embodiment, the condition for stopping the iterative lowering of the detection threshold is fulfilled if three second peaks are identified in four consecutive heart cycles.

In an embodiment, the length of the absolute refractory period is between 200 ms and 500 ms, in particular between 250 ms and 450 ms, in particular between 300 ms and 400 ms after one of the first peaks is detected. Typically, the absolute refractory periods of the human or animal heart lies in a range between 200 ms 500 ms. In this absolute refractory period, no further contraction of the ventricle is to be expected. Thus, if any signal is observed within the previously mentioned time period after one of the first peaks, it is likely not to be an R-wave, but a P-wave or a wave reflecting HIS-bundle activity. Rather, any second peak is to be expected to be detected prior to the first peak, e.g., within a time period between 200 ms and 500 ms, in particular between 250 ms and 450 ms, in particular between 300 ms and 400 ms before one of the first peaks is detected.

In an aspect, the present invention relates to a method for determining an R wave detection threshold of a His electrode channel of an implantable medical device for stimulating human or animal heart. In this context, the implantable medical device for stimulating a human or animal heart complies with the above given explanations of such a device. The method comprises the steps explained the following.

In a first step, an electrical signal (e.g., an ECG) of a human or animal heart is detected with a detection unit of the implantable medical device configured to detect an electrical signal at the His bundle of the human or animal heart. This electrical signal is detected at the His bundle of the human or animal heart to be treated.

Subsequently, first peaks within the electrical signal are identified. In doing so, one assumes that the first peaks are R waves of the electrical signal. This, however, needs to be confirmed by the measures explained in the following.

In a further step, an interval between two consecutive first peaks is determined. Based on a number of such intervals, a mean interval between two consecutive first peaks in each case is determined for first number of pairs of consecutive first peaks.

Depending on the determined interval between two consecutive first peaks, these first peaks are then classified as R waves. To be more precise, two consecutive first peaks are classified as R waves if the interval between these first peaks lies within a range of more than 80 % but less than 120 % of the mean interval. If the interval between two first peaks is bigger than 120 % of the mean interval or lower than 80 % of the mean interval, it is considered that one of the first peaks is an R wave and the other of the first peaks is a ventricular extrasystole or a further cardiac signal other than an R wave.

After at least some of the first peaks have been successfully identified as R waves, the detection threshold of the detection unit is adjusted so as to be able to also detect R waves having a smaller amplitude than the already detected R waves, but to detect at the same time no other cardiac signals than R waves in the analyzed electrical signal of the heart to be treated. For this purpose, the detection threshold of the detection unit is adjusted to allow an exclusive detection of R waves in the electrical signal of the human or animal heart. This adjustment is done by performing the steps explained in the following.

First, an initial detection threshold is defined. In doing so, the detection threshold corresponds in a first variant to an amplitude of a single first peak that has already been successfully classified as R wave. In a second variant, the detection threshold corresponds to a mean amplitude of a plurality of first peaks that have already been successfully classified as R waves. Thus, in this step a rather non-sensitive detection threshold is defined that is appropriate to detect future R waves if the R wave amplitude does not significantly decrease with respect to the R waves already detected within the electrical signal of the human or animal heart.

Subsequently, the detection threshold is iteratively lowered until at least one second peak is identified within the electrical signal. It is to be noted that the second peak is a physiologic different peak than the first peak. For such identification or classification of the second peak, it is necessary that an interval between the at least one second peak and an adjacent first peak is in a range of the length of an absolute refractory period of the heart. According to an embodiment, the second peak is identified within the electrical signal if an interval between the at least one second peak and an adjacent first peak, preferably the preceding first peak, is smaller than or equal to 80 % of the mean interval. Typically, only a P wave or a signal of the His bundle activity can be located within such a small time interval to an R wave. In an embodiment, care is taken that the at least one second peak is observed prior to an adjacent first peak within an interval being in a range of the length of an absolute refractory period of the heart.

If the detection threshold would be left at this level, not only R waves, but also other cardiac signals such as a P wave or a His bundle activity would be observed. However, for proper functioning of the implantable medical device, it is particularly important to detect only R waves in order to have positive knowledge on ventricular activity of the human or animal heart to be stimulated. Therefore, the detection threshold is subsequently increased by a predetermined factor. This increase causes that only first peaks (i.e., suspected R waves) but no second peaks can be identified within the electrical signal any longer.

In an aspect, the present invention relates to computer program product comprising computer-readable code that causes a processor to perform the steps explained in the following when executed on the processor.

In a first step, an electrical signal (e.g., an ECG) of a human or animal heart is detected with a detection unit of the implantable medical device configured to detect an electrical signal at the His bundle of the human or animal heart. This electrical signal is detected at the His bundle of the human or animal heart to be treated.

Subsequently, first peaks within the electrical signal are identified. In doing so, one assumes that the first peaks are R waves of the electrical signal. This, however, needs to be confirmed by the measures explained in the following.

In a further step, an interval between two consecutive first peaks is determined. Based on a number of such intervals, a mean interval between two consecutive first peaks in each case is determined for first number of pairs of consecutive first peaks.

Depending on the determined interval between two consecutive first peaks, these first peaks are then classified as R waves. To be more precise, two consecutive first peaks are classified as R waves if the interval between these first peaks lies within a range of more than 80 % but less than 120 % of the mean interval. If the interval between two first peaks is bigger than 120 % of the mean interval or lower than 80 % of the mean interval, it is considered that one of the first peaks is an R wave and the other of the first peaks is a ventricular extrasystole or a further cardiac signal other than an R wave.

After at least some of the first peaks have been successfully identified as R waves, the detection threshold of the detection unit is adjusted so as to be able to also detect R waves having a smaller amplitude than the already detected R waves, but to detect at the same time no other cardiac signals than R waves in the analyzed electrical signal of the heart to be treated. For this purpose, the detection threshold of the detection unit is adjusted to allow an exclusive detection of R waves in the electrical signal of the human or animal heart. This adjustment is done by performing the steps explained in the following.

First, an initial detection threshold is defined. In doing so, the detection threshold corresponds in a first variant to an amplitude of a single first peak that has already been successfully classified as R wave. In a second variant, the detection threshold corresponds to a mean amplitude of a plurality of first peaks that have already been successfully classified as R waves. Thus, in this step a rather non-sensitive detection threshold is defined that is appropriate to detect future R waves if the R wave amplitude does not significantly decrease with respect to the R waves already detected within the electrical signal of the human or animal heart.

Subsequently, the detection threshold is iteratively lowered until at least one second peak is identified within the electrical signal. It is to be noted that the second peak is a physiologic different peak than the first peak. For such identification or classification of the second peak, it is necessary that an interval between the at least one second peak and an adjacent first peak is in a range of the length of an absolute refractory period of the heart. According to an embodiment, the second peak is identified within the electrical signal if an interval between the at least one second peak and an adjacent first peak, preferably the preceding first peak, is smaller than or equal to 80% of the mean interval. Typically, only a P wave or a signal of the His bundle activity can be located within such a small time interval to an R wave. In an embodiment, care is taken that the at least one second peak is observed prior to an adjacent first peak within an interval being in a range of the length of an absolute refractory period of the heart.

If the detection threshold would be left at this level, not only R waves, but also other cardiac signals such as a P wave or a His bundle activity would be observed. However, for proper functioning of the implantable medical device, it is particularly important to detect only R waves in order to have positive knowledge on ventricular activity of the human or animal heart to be stimulated. Therefore, the detection threshold is subsequently increased by a predetermined factor. This increase causes that only first peaks (i.e., suspected R waves) but no second peaks can be identified within the electrical signal any longer.

In an aspect, the present invention relates to a medical method of treating a human or animal patient in need of such treatment by means of the implantable medical device for stimulating a human or animal heart. In this context, the implantable medical device comprises a processor, a memory unit, a stimulation unit configured to stimulate the His bundle of the human or animal heart, and a detection unit configured to detect electrical signals at the His bundle of the same heart. The method comprises the steps explained in the following.

In a first step, an electrical signal (e.g., an ECG) of a human or animal heart is detected with a detection unit of the implantable medical device configured to detect an electrical signal at the His bundle of the human or animal heart. This electrical signal is detected at the His bundle of the human or animal heart to be treated.

Subsequently, first peaks within the electrical signal are identified. In doing so, one assumes that the first peaks are R waves of the electrical signal. This, however, needs to be confirmed by the measures explained in the following.

In a further step, an interval between two consecutive first peaks is determined. Based on a number of such intervals, a mean interval between two consecutive first peaks in each case is determined for first number of pairs of consecutive first peaks.

Depending on the determined interval between two consecutive first peaks, these first peaks are then classified as R waves. To be more precise, two consecutive first peaks are classified as R waves if the interval between these first peaks lies within a range of more than 80 % but less than 120 % of the mean interval. If the interval between two first peaks is bigger than 120 % of the mean interval or lower than 80 % of the mean interval, it is considered that one of the first peaks is an R wave and the other of the first peaks is a ventricular extrasystole or a further cardiac signal other than an R wave.

After at least some of the first peaks have been successfully identified as R waves, the detection threshold of the detection unit is adjusted so as to be able to also detect R waves having a smaller amplitude than the already detected R waves, but to detect at the same time no other cardiac signals than R waves in the analyzed electrical signal of the heart to be treated. For this purpose, the detection threshold of the detection unit is adjusted to allow an exclusive detection of R waves in the electrical signal of the human or animal heart. This adjustment is done by performing the steps explained in the following.

First, an initial detection threshold is defined. In doing so, the detection threshold corresponds in a first variant to an amplitude of a single first peak that has already been successfully classified as R wave. In a second variant, the detection threshold corresponds to a mean amplitude of a plurality of first peaks that have already been successfully classified as R waves. Thus, in this step a rather non-sensitive detection threshold is defined that is appropriate to detect future R waves if the R wave amplitude does not significantly decrease with respect to the R waves already detected within the electrical signal of the human or animal heart.

Subsequently, the detection threshold is iteratively lowered until at least one second peak is identified within the electrical signal. It is to be noted that the second peak is a physiologic different peak than the first peak. For such identification or classification of the second peak, it is necessary that an interval between the at least one second peak and an adjacent first peak is in a range of the length of an absolute refractory period of the heart. According to an embodiment, the second peak is identified within the electrical signal if an interval between the at least one second peak and an adjacent first peak, preferably the preceding first peak, is smaller than or equal to 80% of the mean interval. Typically, only a P wave or a signal of the His bundle activity can be located within such a small time interval to an R wave. In an embodiment, care is taken that the at least one second peak is observed prior to an adjacent first peak within an interval being in a range of the length of an absolute refractory period of the heart.

If the detection threshold would be left at this level, not only R waves, but also other cardiac signals such as a P wave or a His bundle activity would be observed. However, for proper functioning of the implantable medical device, it is particularly important to detect only R waves in order to have positive knowledge on ventricular activity of the human or animal heart to be stimulated. Therefore, the detection threshold is subsequently increased by a predetermined factor. This increase causes that only first peaks (i.e., suspected R waves) but no second peaks can be identified within the electrical signal any longer.

If no first peaks are identified within a predetermined number of mean intervals between two consecutive first peaks, the human or animal heart is stimulated by applying a stimulation pulse with the stimulation unit. For instance, the His bundle of the heart is stimulated. The predetermined number of mean intervals can be, e.g., 1, 2, 3, 4, 5, 6, 7, 8, 9, 10, 15, 20, 25, or 30. If the human heart has not shown any intrinsic activity over such period of time, a His bundle pacing is necessary to avoid severe effects of the missing intrinsic heart activity.

A further aspect of the present invention relates to a test for adjusting the stimulation amplitude of a stimulation unit of an implantable medical device configured to be used for His bundle pacing, e.g., the stimulation unit of the above-described implantable medical device. In this context, the stimulation amplitude of the His bundle electrode is successively lowered, wherein the time between application of a stimulation pulse and the detection of an intrinsic cardiac event with the His bundle electrode is detected in each step. Considering that only a single R wave can be detected within each heart cycle and considering that the intrinsic conduction time from a P wave or from an atrial stimulation, respectively, to the R wave is longer than the time between an effective stimulus at the His bundle and a subsequent R wave it can be decided which minimal value of the stimulation amplitude is necessary for an effective His bundle pacing.

Starting from this minimum stimulation amplitude, the morphology of the signal detected with the His bundle electrode can be evaluated in a second test phase. In doing so, the limit between the stimulation threshold for selective His bundle pacing and the stimulation threshold for non-selective His bundle pacing can be determined.

For this second test phase, the stimulation amplitude is successively increased until a significant change of the signal morphology is detected. The stimulation amplitude being effective at the time of the change can then be interpreted as limiting value between selective His bundle pacing and non-selective His bundle pacing.

Since the limiting value is patient-specific and since the limiting value can be particularly reliably determined with a 12-channel electrocardiogram (ECG), it is appropriate to perform this second phase of the test during a follow-up examination by assessing the cardiac state of the patient with a 12-channel ECG. Afterwards, the obtained information can be entered and stored in the implantable medical device so that it can be used for subsequent regular automatic measurements.

This aspect of the invention enables determining a minimum stimulation amplitude and enables a differentiation between selective and non-selective His bundle pacing. The signal morphology can be particularly well assessed by evaluating at least one of the maximum value, the minimum value, the width (such as the full width at half maximum) and zero crossings of the detected and evaluated signal.

All aspects and embodiments of the described implantable medical device can be combined in any desired way and can be transferred either individually or in any arbitrary combination to the described methods and to the described computer program product. Likewise, all details and embodiments of the described methods can be combined in any desired way and can be transferred individually or in any arbitrary combination to the respective other method, to the described implantable medical device and to the described computer program product. Finally, all variants and embodiments of the described computer program product can be combined in any desired way and can be transferred individually or in any arbitrary combination to the described implantable medical device or to the described methods.

Further details of aspects of the present invention will be explained with respect to exemplary embodiments and accompanying Figures. In the Figures:
- Figure 1: shows a first schematic depiction of a simulation of an electrocardiogram detected with a His bundle electrode along with its evaluation and
- Figure 2: shows two plots of the same schematic depiction of a simulated electrocardiogram with different detection thresholds along with evaluations of the electrocardiogram.

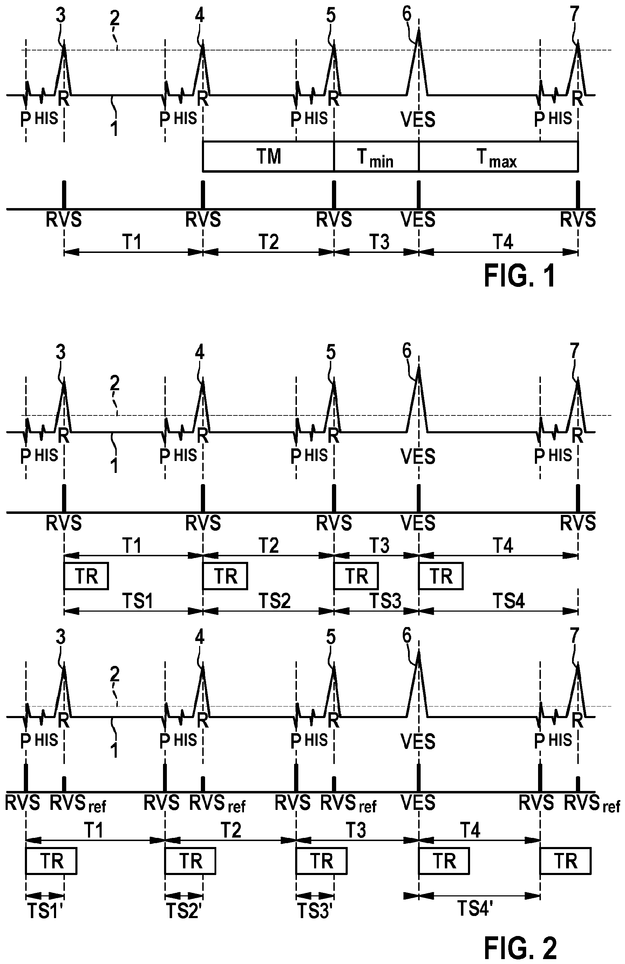

Figure 1 shows a simulated electrocardiogram 1 comprising a plurality of signals to be assigned to a P wave (denoted with P), to His bundle activity (denoted with HIS) and to an R wave (denoted with R). Furthermore, a ventricular extrasystole VES is indicated in the electrocardiogram.

A detection threshold 2 of a His electrode by which the electrocardiogram 1 is detected is set to extend along the amplitudes of the R waves of the electrocardiogram 1. Thus, the detection threshold 2 is comparatively non-sensitive.

Each R wave corresponds to a first peak in the electrocardiogram 1 that is classified to be a right ventricular systole RVS. A first interval T1 between a first R wave 3 and a second R wave 4 is as long as a second interval T2 between the second R wave 4 and a third R wave 5. Since the first interval T1 and the second interval T2 have an identical duration, a mean heart cycle duration or mean interval TM between two consecutive R waves corresponds to T1 and T2.

A third interval T3 between the third R wave 5 and a ventricular extrasystole 6 is significantly shorter than the mean interval TM between two consecutive R waves. This third interval T3 can also be denoted as minimum heart cycle duration Tmin. It is shorter than 80 % of the mean interval TM between two consecutive R waves.

In contrast, a subsequent forth interval T4 between the ventricular extrasystole 6 and a fourth R wave 7 is significantly longer than the mean interval TM between two consecutive R waves. The fourth interval T4 can also be denoted as maximum heart cycle duration Tmax. It lasts longer than 120 % of the mean interval TM between two consecutive R waves. The minimum heart cycle duration Tmin and the maximum heart cycle duration Tmax are not considered for calculating the mean interval TM between two consecutive R waves for updating this mean interval TM. If the minimum heart cycle duration Tmin and the maximum heart cycle duration Tmax were considered, this would distort proper calculation of the mean interval TM between two consecutive R waves.

The first time interval T1 and the second time interval T2 reflect the duration of a heart cycle which starts at a first R wave (e.g. the first R wave 3) and ends at the next adjacent R wave (e.g., the second R wave 4).

Since the detection threshold 2 is set to a comparatively high value (i.e., it is a non-sensitive detection threshold), only R waves are detected within the electrocardiogram 1. Consequently, the evaluation of the electrocardiogram 1 depicted below the electrocardiogram 1 only shows right ventricular systoles RVS and the ventricular extrasystole VES, but no detections of a P wave or of a His bundle specific signal.

Figure 2 shows the same electrocardiogram 1 like Figure 1. Furthermore, the same numeral references are used as in Figure 1 for explaining the same or similar elements. The only difference between Figure 2 and Figure 1 is the lowered detection threshold 2. In the upper panel of Figure 2, the detection threshold 2 is still set to such a value that only R waves are detected so that only right ventricular systoles RVS and the ventricular extrasystole VES are detected in the according evaluation line.

A first sensing time TS1 between a first right ventricular systole and a second right ventricular systole is equally long than the first time interval T1 and thus significantly longer than an absolute refractory period TR. Likewise, a second sensing time TS2 equals the second time interval T2, a third sensing time TS3 equals the third time interval T3 and a fourth sensing time TS4 equals the fourth time interval T4. The second sensing time TS2, the third sensing time TS3 and the fourth sensing time TS4 are each longer than the absolute refractory period TR.

In the lower panel of Figure 2, the detection threshold 2 is further decreased. Now, not only R waves 3, 4, 5, 7 and the ventricular extrasystole 6, but also the P waves of the electrocardiogram 1 are detected and evaluated. As indicated in the evaluation line of the lower panel of Figure 2, the peak of the P wave is erroneously evaluated to be a right ventricular systole RVS. However, in this case, the real right ventricular systole (denoted as RVSref in the lower panel of Figure 2) is detected with a sensing time TS1' being shorter than the absolute refractory period TR and also shorter than the first interval T1. Also in the second and third heart cycles, a second sensing time TS2' and a third sensing time TS3' are smaller than the absolute refractory period TR. This is, in the embodiment shown in Figure 2, the relevant criterion to identify the detected cardiac signals as second-type cardiac signals (i.e., second peaks). This means that the detection threshold 2 is presently set to a too low value so that the detection is now done too sensitive. Consequently, it is necessary to increase the detection threshold 2 once again in order to only detect R waves in the electrocardiogram 1.

Even though the fourth sensing time TS4' is longer than the absolute refractory period TR, this does not impair the finding that the detection threshold 2 is set to a too low value in the lower panel of Figure 2. Rather, the occurrence of additional cardiac signals in three out of four measurements is fully sufficient in this embodiment to indicate that the detection threshold 2 needs to be increased again.

It should be noted that Figure 2 makes reference to the absolute refractory period TR. According to an embodiment, a classification of individual peaks in the electrocardiogram 1 to first peaks or R waves on the one hand and to second peaks or other cardiac signals on the other hand can be fully done without considering the absolute refractory period TR. In that case, it is sufficient to compare the sensing times TS1, TS2, TS3 and TS4 with the first interval T1, the second interval T2, the third interval T3 and the fourth interval T4. If the respective sensing time is shorter than 80 % of the corresponding time interval, this is an indicator that any additionally detected signals cannot be additional R waves, but are rather considered to be cardiac signals other than R waves. In such a case, the detection threshold 2 needs to be increased again to be set to such a level at which a detection of only R waves (with a very high sensitivity) is achieved.

## Claims

1. Implantable medical device for stimulating a human or animal heart, comprising a processor, a memory unit, a stimulation unit configured to stimulate the His bundle of a human or animal heart, and a detection unit configured to detect an electrical signal at the His bundle of the same heart,
**characterized**
**in that** the memory unit comprises a computer-readable program that causes the processor to perform the following steps when executed on the processor:
a) detecting, with the detection unit, an electrical signal (1) of the human or animal heart;
b) identifying first peaks (3, 4, 5, 6, 7) within the electrical signal, the first peaks being suspected to be R waves (3, 4, 5, 7);
c) determining a mean interval (TM) between two consecutive first peaks in each case (3,4; 4,5) for a first number of pairs of consecutive first peaks (3,4; 4,5);
d) classifying two consecutive first peaks (3,4; 4,5) as R waves if an interval (T1, T2) between these two consecutive first peaks (3,4; 4,5) is bigger than 80 % and smaller than 120 % of the mean interval (TM);
e) adjusting a detection threshold (2) of the detection unit so as to allow the detection unit to exclusively detect first peaks (3, 4, 5, 6, 7) in the electrical signal (1) of the human or animal heart, wherein the following steps are performed for adjusting the detection threshold (2) of the detection unit:
i) defining a detection threshold (2) corresponding to a) an amplitude of a single first peak (3, 4, 5, 7) that has been successfully classified as R wave or b) a mean amplitude of a plurality of first peaks (3, 4, 5, 7) that have been successfully classified as R wave;
ii) iteratively decreasing the detection threshold (2) until at least one second peak is identified within the electrical signal (1), wherein an interval (TS1', TS2', TS3') between the at least one second peak and an adjacent first peak (3, 4, 5) is in a range of the length of an absolute refractory period of the heart;
iii) increasing the detection threshold (2) by a predetermined factor, wherein the increasing causes that only first peaks (3, 4, 5, 6, 7) but no second peaks can be identified within the electrical signal (1).

2. Implantable medical device according to claim 1, **characterized in that** the computer-readable program causes the processor not to use an interval (T3, T4) between two consecutive first peaks (5,6; 6,7) for determining the mean interval between two consecutive first peaks, if the interval (T3, T4) is a) smaller than or equal to 80 % or b) bigger than or equal to 120 % of at least two other intervals (T1, T2) between two consecutive first peaks (3,4; 4,5).

3. Implantable medical device according to claim 1 or 2, **characterized in that** the computer-readable program causes the processor to iteratively decrease the detection threshold (2) until at least one second peak is identified within the electrical signal (1), wherein the second peak has an amplitude which is lower than the amplitude of the single first peak (3, 4, 5, 7) that has been successfully classified as R wave or the mean amplitude of the plurality of first peaks (3, 4, 5, 7) that have been successfully classified as R wave.

4. Implantable medical device according to one of the claims 1 to 3, **characterized in that** the computer-readable program causes the processor not to use two consecutive intervals (T3, T4) between two consecutive first peaks (5,6; 6,7) in each case for determining the mean interval (TM) between two consecutive first peaks (3,4; 4,5), if a first of the two consecutive intervals (T3) is smaller than or equal to 80 % of at least two other intervals (T1, T2) between two consecutive first peaks (3,4; 4,5) and if a second (T4) of the two consecutive intervals is bigger than or equal to 120 % of at least two other intervals (T1, T2) between two consecutive first peaks (3,4; 4,5).

5. Implantable medical device according to any of the preceding claims, **characterized in that** the computer-readable program causes the processor to continuously update the mean interval (TM) between two consecutive first peaks (3,4; 4,5).

6. Implantable medical device according to claim 5, **characterized in that** the computer-readable program causes the processor not to use an interval (T3, T4) between two consecutive first peaks (5,6; 6,7) for updating the mean interval (TM) between two consecutive first peaks (3,4; 4,5), if the interval is a) smaller than or equal to 80 % or b) bigger than or equal to 120 % of the current mean interval (TM) between two consecutive first peaks (3,4; 4,5).

7. Implantable medical device according to any of the preceding claims, **characterized in that** the predetermined factor serves for an increase of the detection threshold (2) to a level corresponding to a level prior to the last decrease of the detection threshold (2) during the iterative decrease of the detection threshold (2).

8. Implantable medical device according to any of the preceding claims, **characterized in that** the predetermined factor is at least 1.1 of an absolute voltage of the detection threshold (2).

9. Implantable medical device according to any of the preceding claims, **characterized in that** the computer-readable program causes the processor to stop the iterative lowering of the detection threshold (2) if a first number of second peaks is identified within the electrical signal (1) in a second number of consecutive heart cycles, the first number being smaller than the second number.

10. Implantable medical device according to any of the preceding claims, **characterized in that** the length of the absolute refractory period is 200ms to 500ms.

11. Method for determining an R wave detection threshold of a His electrode channel of an implantable medical device for stimulating a human or animal heart according to any of the preceding claims, the method comprising the following steps:
a) detecting, with a detection unit configured to detect an electrical signal at the His bundle of a human or animal heart, an electrical signal (1) at the His bundle of a human or animal heart;
b) identifying first peaks (3, 4, 5, 6, 7) within the electrical signal, the first peaks (3, 4, 5, 6, 7) being suspected to be R waves (3, 4, 5, 7);
c) determining a mean interval (TM) between two consecutive first peaks in each case (3,4; 4,5) for a first number of pairs of consecutive first peaks (3,4; 4,5);
d) classifying two consecutive first peaks (3,4; 4,5) as R waves if an interval (T1, T2) between these two consecutive first peaks (3,4; 4,5) is bigger than 80 % and smaller than 120 % of the mean interval (TM);
e) adjusting a detection threshold (2) of the detection unit so as to allow the detection unit to exclusively detect first peaks (3, 4, 5, 6, 7) in the electrical signal (1) of the human or animal heart, wherein the following steps are performed for adjusting the detection threshold (2) of the detection unit:
i) defining a detection threshold (2) corresponding to a) an amplitude of a single first peak (3, 4, 5, 7) that has been successfully classified as R wave or b) a mean amplitude of a plurality of first peaks (3, 4, 5, 7) that have been successfully classified as R wave;
ii) iteratively decreasing the detection threshold (2) until at least one second peak is identified within the electrical signal (1), wherein an interval (TS1', TS2', TS3') between the at least one second peak and an adjacent first peak (3, 4, 5) in a range of the length of an absolute refractory period of the heart;
iii) increasing the detection threshold (2) by a predetermined factor, wherein the increasing causes that only first peaks (3, 4, 5, 6, 7) but no second peaks can be identified within the electrical signal (1).

12. Computer program product comprising computer-readable code that causes a processor to perform the following steps when executed on the processor:
a) detecting, with a detection unit configured to detect an electrical signal (1) at the His bundle of a human or animal heart, an electrical signal at the His bundle of a human or animal heart;
b) identifying first peaks (3, 4, 5, 6, 7) within the electrical signal, the first peaks being suspected to be R waves (3, 4, 5, 7);
c) determining a mean interval (TM) between two consecutive first peaks in each case (3,4; 4,5) for a first number of pairs of consecutive first peaks (3,4; 4,5);
d) classifying two consecutive first peaks (3,4; 4,5) as R waves if an interval (T1, T2) between these two consecutive first peaks (3,4; 4,5) is bigger than 80 % and smaller than 120 % of the mean interval (TM);
e) adjusting a detection threshold (2) of the detection unit so as to allow the detection unit to exclusively detect first peaks (3, 4, 5, 6, 7) in the electrical signal (1) of the human or animal heart, wherein the following steps are performed for adjusting the detection threshold (2) of the detection unit:
i) defining a detection threshold (2) corresponding to a) an amplitude of a single first peak (3, 4, 5, 7) that has been successfully classified as R wave or b) a mean amplitude of a plurality of first peaks (3, 4, 5, 7) that have been successfully classified as R wave;
ii) iteratively decreasing the detection threshold (2) until at least one second peak is identified within the electrical signal (1), wherein an interval (TS1', TS2', TS3') between the at least one second peak and an adjacent first peak (3, 4, 5) is in a range of the length of an absolute refractory period of the heart;
iii) increasing the detection threshold (2) by a predetermined factor, wherein the increasing causes that only first peaks (3, 4, 5, 6, 7) but no second peaks can be identified within the electrical signal (1).

13. Method of treatment of a human or animal patient in need of such treatment by means of an implantable medical device for stimulating a human or animal heart, wherein the implantable medical device comprises a processor, a memory unit, a stimulation unit configured to stimulate the His bundle of a human or animal heart, and a detection unit configured to detect an electrical signal at the His bundle of the same heart, the method comprising the following steps:
a) detecting, with the detection unit, an electrical signal (1) of the human or animal heart;
b) identifying first peaks (3, 4, 5, 6, 7) within the electrical signal, the first peaks being suspected to be R waves (3, 4, 5, 7);
c) determining a mean interval (TM) between two consecutive first peaks (3,4; 4,5) in each case for a first number of pairs of consecutive first peaks (3,4; 4,5);
d) classifying two consecutive first peaks (3,4; 4,5) as R waves if an interval (T1, T2) between these two consecutive first peaks is bigger than 80 % and smaller than 120 % of the mean interval (TM);
e) adjusting a detection threshold (2) of the detection unit so as to allow the detection unit to exclusively detect first peaks (3, 4, 5, 6, 7) in the electrical signal of the human or animal heart, wherein the following steps are performed for adjusting the detection threshold (2) of the detection unit:
i) defining a detection threshold (2) corresponding to a) an amplitude of a single first peak (3, 4, 5, 7) that has been successfully classified as R wave or b) a mean amplitude of a plurality of first peaks (3, 4, 5, 7) that have been successfully classified as R wave;
ii) iteratively decreasing the detection threshold (2) until at least one second peak is identified within the electrical signal (1), wherein an interval (TS1', TS2', TS3') between the at least one second peak and an adjacent first peak (3, 4, 5) is in a range of the length of an absolute refractory period of the heart;
iii) increasing the detection threshold (2) by a predetermined factor, wherein the increasing causes that only first peaks (3, 4, 5, 6, 7) but no second peaks can be identified within the electrical signal;
f) stimulating the human or animal heart by applying a stimulation pulse with the stimulation unit if no first peaks (3, 4, 5, 6, 7) are identified within a predetermined number of mean intervals (TM) between two consecutive first peaks.

14. Method of treatment according to claim 13, **characterized in that** the stimulation of the human or animal heart is a stimulation of the HIS-bundle, wherein the His bundle is only stimulated with a stimulation pulse if no first peaks (3, 4, 5, 7) classified as R waves are identified within a predetermined number of mean intervals (TM) between two consecutive first peaks.

15. Method of treatment according to claim 13 or 14, **characterized in that** adjusting the detection threshold (2) of the detection unit further comprises the steps of:
iteratively decreasing the detection threshold (2) until at least one second peak is identified within the electrical signal (1), wherein the second peak has an amplitude which is lower than the amplitude of the single first peak (3, 4, 5, 7) that has been successfully classified as R wave or the mean amplitude of the plurality of first peaks (3, 4, 5, 7) that have been successfully classified as R wave.
